Europäisches Patentamt

European Patent Office

Office européen des brevets

(19)

(11) Publication number: **0 318 487 B1**

# EUROPEAN PATENT SPECIFICATION

(12)

(45) Date of publication of patent specification: **13.10.93**

(51) Int. Cl.5: **C12N 5/00**, C12N 5/02, C12N 15/00

(21) Application number: **87905144.9**

(22) Date of filing: **04.08.87**

(86) International application number:
**PCT/AU87/00248**

(87) International publication number:
**WO 88/00967 (11.02.88 88/04)**

(54) SERUM FREE TISSUE CULTURE MEDIUM CONTAINING POLYMERIC CELL-PROTECTIVE AGENT.

(30) Priority: **04.08.86 AU 7266/86**

(43) Date of publication of application:
**07.06.89 Bulletin 89/23**

(45) Publication of the grant of the patent:
**13.10.93 Bulletin 93/41**

(84) Designated Contracting States:
**AT BE CH DE FR GB IT LI LU NL SE**

(56) References cited:
EP-A- 0 248 656    AU-A- 2 232 683
AU-A- 3 857 185    AU-B- 5 445 680
AU-B- 7 526 981    DE-A- 1 226 744
FR-A- 2 527 448    FR-A- 2 559 499
US-A- 4 390 623

Derwent abstract accession n 87-133257/19, class B04 & JP A 2074284 (Teijin KK), 6 April 1987

(73) Proprietor: **THE UNIVERSITY OF NEW SOUTH WALES**
**221-227 Anzac Parade**
**Kensington, NSW 2033(AU)**

Proprietor: **GARVAN INSTITUTE OF MEDICAL RESEARCH**
**384 Victoria Street**
**Darlinghurst, NSW 2010(AU)**

(72) Inventor: **GRAY, Peter, Philip**
**74 Cameron Street**
**Edgecliff, NSW 2027(AU)**
Inventor: **LAZARUS, Leslie**
**5 Woodward Place**
**St. Ives, NSW 2075(AU)**
Inventor: **MARSDEN, Warwick, Lloyd**
**109 Alsion Road**
**Randwick, NSW 2031(AU)**

EP 0 318 487 B1

**Developments in biological standardization v 55, 1983, published 1984 by Karger (Basel), A. Mizrahi "Oxygen in human lymphoblastoid cell line cultures and effect of polymers in agitated and aerated cultures", pages 93-102**

(74) Representative: **Perry, Robert Edward et al GILL JENNINGS & EVERY, Broadgate House, 7 Eldon Street London EC2M 7LH (GB)**

## Description

The present invention relates to an improved culture medium for use in culturing mammalian cells requiring attachment, under aeration.

The present invention further relates to a method for producing polypeptides from mammalian cell lines into which DNA coding for the desired polypeptide has been inserted.

Developments in recombinant DNA technology have made it possible to insert DNA coding for a desired polypeptide into the genome of continuous mammalian cell lines. These cell lines have certain advantages over the use of prokaryotic hosts such as E. coli. These advantages include the ability to secrete and glycosylate proteins, and the ability to process genetic material in an identical fashion to that occurring in cells in the body. To be weighed against these advantages is the fact that the use of recombinant mammalian cells for the production of proteins involves large scale tissue culture, mostly in antibiotic free medium. Tissue culture requires more precise environmental control and more rigorous design and operation of equipment to avoid contamination than does prokaryotic cell culture. A further disadvantage in the use of mammalian cells is that the medium used in the cultivation of these cells typically contains serum. The presence of serum greatly adds to the complexity of isolation and purification of the desired polypeptide product from the culture media.

## BACKGROUND ART

It is possible to place cells used in tissue culture in two broad categories: (1) Those which grow free in suspension culture, such as hybridoma cells, and (2) those which grow attached to surfaces. The former cells can be grown in suspension culture providing consideration is taken of the fact that the cells do not possess cell walls and are hence sensitive to shear forces. Cells in the second category require increased surface area of cell growth in order to scale-up production. Various devices have been described in the literature to provide this increased surface area, including devices providing glass honeycomb surfaces, surfaces wrapped as sheets and glass spheres of several mm diameter. A full review of the various approaches described in the literature appears in the paper by Glacken et al (1983), Trends in Biotechnology, Vol. 1, 102-108.

Another approach is to use smaller diameter spherical particles which can be constructed from a range of materials including cross linked dextran, polystyrene or glass. The advantage of these particles, sometimes referred to as microcarriers, is that it is possible to achieve very high surface areas per unit mass of beads (5000 $cm^2$/gm) with particles with diameters of the order of 100-200 um. An extension of this approach is the use of porous microcarriers which allow the cells to grow inside the beads as well as on the outer surface. Another possibility is to use even smaller diameter latex beads (of the order of 1 um diameter) to act as nucleation sites for the attachment of cells to these beads and to each other. Under certain conditions it may be possible to induce the cells to attach to each other to form agglomerates of cells or flocs. Once cells are attached to either microcarriers or beads or flocs, it is possible to grow the cells in suspension culture. Equipment for growing cells in suspension culture is in routine use and the use of this equipment avoids the problem of developing technology using different equipment such as membrane devices. One of the difficulties encountered, however, with cells grown on microcarriers is that at the cell densities achieved oxygen limitation may occur. While such a limitation may be overcome using known aeration techniques such aeration may cause the cells to be disassociated from the support medium, then the culture medium is serum-free.

## DISCLOSURE OF THE INVENTION

The present inventors have developed a novel tissue culture medium, which enables the aeration of cultures of cells attached to a support medium, without the consequent detachment of the cells in the absence of serum. In addition, the present inventors have developed novel methods for the cultivation of cells requiring attachment.

The present invention consists in a method of culturing mammalian cells which require attachment comprising the following steps:

1. Culturing the mammalian cells in a serum containing medium in a reaction vessel provided with a support medium to which the cells will attach until the cells have attached thereto and grown to the desired cell concentration;

2. Decanting the serum containing medium;

3. Replacing the decanted medium with a serum-free medium which includes in solution a non-toxic polymer which is capable of acting as a cell protective agent by reducing film drainage around the cells and the support medium; and

4. Subjecting the culture to direct aeration.

Without wishing to be bound by scientific theory it is believed that the polymer which acts as a cell protective agent prevents the disassociation of the cells from the support medium and lysis of the cells, when the cells are exposed to direct aeration in the absence of serum, by forming a protective film around the cell/support agglomerate. It is believed that this protective film around the cell/support agglomerate prevents the film of culture medium which surrounds the cells from draining away when the cell/support agglomerate comes into contact with air bubbles. This draining of culture medium is referred to as film drainage. It is also believed that this film may provide a barrier which protects the cells from physical damage caused by the air bubbles.

The term "direct aeration" is used to describe aeration of the culture medium in which the cultured cells are exposed to gas bubbles.

It is preferred that the polymer is non-ionic and low foaming.

In a preferred embodiment of the present invention the polymer is either polyethylene glycol, polyvinyl pyrollidone or a polymer containing one or more alkylene oxides. It is preferred that the alkylene oxides contain two or three carbon atoms. It is particularly preferred that the polymer is a copolymer of propylene oxide and ethylene oxide.

In a further preferred embodiment of the present invention the polymer of alkylene oxides is present in a concentration of 0.1 to 1.0%.

In another preferred embodiment of the present invention the tissue culture medium is essentially protein-free.

In a preferred embodiment of the present invention the reaction vessel is divided into a culture region and a cell-free region by means of a filter through which culture medium but not cells can pass.

Aeration of the culture medium may be achieved by bubbling a gas into the cell-free region of the reactor vessel. This method of aeration will lead to exposure of the cultured cells to gas bubbles as a proportion of the bubbles will generally pass through the filter means. However, it is presently preferred that the aeration is conducted by using an air lift positioned within the reaction vessel.

In preferred embodiments of the present invention the mammalian cells are a continuous cell line, particularly Chinese hamster ovary cell line, into which DNA coding for a desired polypeptide, particularly human growth hormone (hGH), has been inserted.

Preferably the DNA coding the desired polypeptide is under the control of an inducible promoter, which is induced by a component of the essentially serum-free medium. Further, in a preferred embodiment the essentially serum-free medium is essentially protein-free.

The agitation of the culture may be achieved using an impellor such as an anchor type impellor at a rate at which shear forces generated are low. Obviously other impellor configurations can be used providing the shear forces generated are low.

In a further preferred embodiment the essentially serum-free medium is added continuously and the desired polypeptide recovered continuously.

In order that the nature of the invention may be better understood preferred forms thereof are hereinafter described by way of example, with reference to the accompanying drawing which shows a representation of a reactor system in which aeration is by means of an air lift positioned within the reaction vessel.

Example 1

A Chinese hamster ovary (CHO) cell line was transfected with DNA coding for human growth hormone linked to a strong regulatable promoter/enhancer sequence. Further details of the genetic construction are given in WO 86/04920.

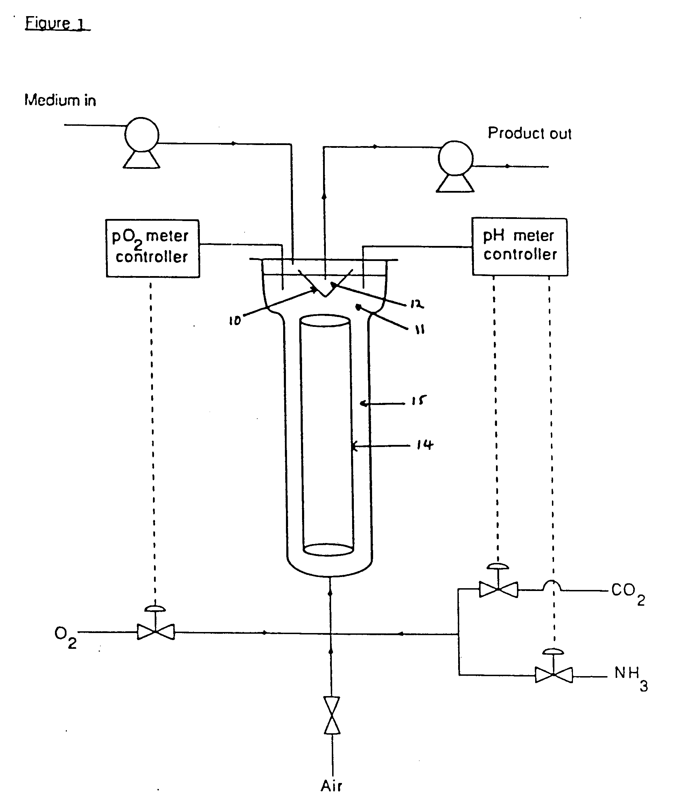

Cells were stored in liquid nitrogen ampoules, then reconstituted by growing in either Roux flasks or roller bottles. Cells were harvested using EDTA, washed in PBS and contacted with pretreated microcarriers. The microcarriers were either dextran based (Cytodex 1 and 2, Pharmacia or polystyrene based, Biosilan, Nunc). Seeding densities of 6-10 cells per carrier were found suitable and resulted in the cells attaching and growing with minimum lag and doubling times of approximately 18 hrs. The medium was a synthetic medium containing 5 or 10% FCS. To build up the number of cells needed to inoculate larger scale vessels, it was possible to add fresh microcarriers to populations of confluent microcarriers and colonisation of the fresh carriers would occur. The desired inoculum of microcarriers was transferred to the configuration of the fermentor shown in Fig. 1.

This example makes use of the air lift principle, whereby a gas phase is used to promote low shear mixing as well as providing the gas phase for mass transfer. A gas phase is bubbled into the bottom of one section of the reactor, (the riser section), which is connected at the top of the reactor to the downcomer section. The introduction of the gas phase into the riser section lowers the density in this section, resulting in the de-aerated higher density culture in the downcomer displacing the lower density culture hence promoting gentle mixing in the reactor. It is possible to join the downcomer and riser sections in several reactor configurations. One configuration, having the riser as a concentric tube 14 inside a cylindrical reaction vessel 15 is shown in the drawing. This configuration has been used previously for the growth of hybridoma cells. It has been shown that it is possible to use the configuration for growth and, product formation by CHO cells attached to microcarriers with gas rates as low as 0.02 VVM. The gas phase used is basically air, which can be supplemented using the usual control methods with carbon dioxide or ammonia, to maintain culture pH. If necessary, oxygen can also be added to the gas phase to maintain the desired level of dissolved oxygen without using excessively high gassing rates. At the low gassing rates used in this work, foaming problems are minimal; however if foaming occurs in the initial stages, e.g. when the cells are growing on serum containing medium, it is possible to control it with the metered addition (using a conductivity sensor or timed addition) of a silicone based antifoam e.g. antifoam C, or other antifoams if they do not interfere with cell growth or product formation. Once the growth stage has occurred and the microcarrier beads are confluent, the cells are washed in 1 volume of PBS, using a stainless steel mesh to maintain the microcarriers. The reactor is then filled with essentially serum-free medium containing a copolymer of propylene oxide and ethylene oxide (Pluriol PE 6800) for the production of hGH. This medium consisted of the following:

| INORGANIC SALTS | mg/l |
|---|---|
| $CaCl_2$ (anhyd.) | 162.15 |
| $CuSO_4.5H_2O$ | 0.0013 |
| $Fe(NO_3)_3.9H_2O$ | 0.05 |
| $FeSO_4.7H_2O$ | 0.417 |
| KCl | 352.5 |
| $KH_2PO_4$ | 30.62 |
| $MgCl_2$ (anhyd.) | 23.33 |
| $MgSO_4$ (anhyd.) | 61.48 |
| NaCl | 6958.5 |
| $NaHCO_3$ | 1338 |
| $NaH_2PO_4(H_2O)$ | 62.5 |
| $ZnSO_4.7H_2O$ | 0.432 |
| $Na_2HPO_4.7H_2O$ | 125 |
| AMINO ACIDS | |
| L-Alanine | 9.0 |
| L-Asparagine.$H_2O$ | 15.0 |
| L-Arginine.HCl | 253.0 |
| L-Aspartic acid | 13.0 |
| L-Cysteine HCl.$H_2O$ | 35.13 |
| L-Cystine.2HCl | 31.29 |

5

| | |
|---|---|
| L-Glutamic acid | 15.00 |
| L-Glutamine | 438.0 |
| Glycine | 23.0 |
| L-histidine HCl.$H_2O$ | 42.0 |
| L-Isoleucine | 56.4 |
| L-Leucine | 65.6 |
| L-Lysine HCl | 109.5 |
| L-Methionine | 19.5 |
| L-Phenylalanine | 38.0 |
| L-Proline | 35.0 |
| L-Serine | 31.5 |
| L-Threonine | 59.4 |
| L-Tryptophan | 10.0 |
| L-Tyrosine (disodium salt) | 59.83 |
| L-Valine | 58.7 |
| VITAMINS | |
| Ascorbic acid | 7.5 |
| Biotin | 0.0037 |
| D-Ca pantothenate | 2.119 |
| Choline chloride | 8.98 |
| Folic acid | 2.66 |
| i-Inositol | 12.61 |
| Nicotinamide | 2.02 |
| Pyridoxal HCl | 2.03 |
| Riboflavin | 0.22 |
| Thiamine HCl | 2.169 |
| Vitamin $B_{12}$ | 0.68 |
| OTHER COMPONENTS | |
| D-Glucose | 3151 |
| HEPES | 3570 |
| Sodium pyruvate | 110 |
| Hypoxanthine (disodium salt) | 2.7 |
| Linoleic acid | 0.045 |
| Putrecine 2HCl | 0.081 |
| Thioctic acid (Lipoic) | 0.103 |
| Thymidine | 0.35 |
| Pluriol PE 6800 | 2000 |

With a cell concentration of $10^7$ cells/ml, it was possible to run the reactor in a continuous fashion and produce a product stream at a dilution rate of 0.15 $hr^{-1}$ and an hGH concentration of 80 mg/1. The reactor could also be run in the fed batch or repeated batch mode, where part or all of the contents could be removed from the reactor via the stainless steel mesh, and fresh media replaced either in a batch or continuous fashion. Using this technique, it was possible to obtain product stream with hGH concentrations

6

up to 250 mg/litre.

In this example it is possible to bubble a gas directly in the culture medium as the medium contains the non-toxic, low-foaming, non-ionic compound Pluriol PE 6800. This compound is obtained by the copolymerization of propylene oxide and ethylene oxide. The presence of this compound in the culture medium enables aeration without causing the cells to disassociate from the support medium.

**Claims**

1. A method of culturing mammalian cells which require attachment, comprising the following steps:

    1. Culturing the mammalian cells in a serum-containing medium in a reaction vessel provided with a support medium to which the cells will attach until the cells have attached thereto and grown to the desired cell concentration;

    2. Decanting the serum-containing medium;

    3. Replacing the decanted medium with a serum-free medium which includes, in solution, a non-toxic polymer which reduces film drainage around the cells and the support medium; and

    4. Subjecting the culture to direct aeration.

2. A method as claimed in claim 1 in which the non-toxic polymer present in the serum-free medium is selected from the group comprising polyethylene glycol and polyvinyl pyrrolidone.

3. A method as claimed in claim 1 in which the non-toxic polymer present in the serum-free medium is a polymer which contains one or more alkylene oxides.

4. A method as claimed in claim 3 in which the alkylene oxide contains 2 or 3 carbon atoms.

5. A method as claimed in claim 4 in which the polymer is a copolymer of propylene oxide and ethylene oxide.

6. A method as claimed in claim 3 in which the polymer of alkylene oxides is present in the medium in a concentration of 0.1 to 1.0% by weight.

7. A method as claimed in any preceding claim in which the reaction vessel is divided into a culture region and a cell-free region, the culture region being separated from the cell-free region by means of a filter through which culture medium but not cells may pass.

8. A method as claimed in claim 7 in which the aeration of the culture is conducted by bubbling a gas into the cell-free region of the reaction vessel.

9. A method as claimed in any preceding claim in which the aeration is carried out by means of a gas lift present within the reaction vessel.

10. A method as claimed in any preceding claim in which the mammalian cell is genetically engineered to contain DNA encoding a polypeptide, such that growth of the cells results in production of the polypeptide.

11. A method as claimed in claim 10 in which the polypeptide is recovered from culture medium continuously removed from the reaction vessel, and fresh serum-free medium is continuously added to the reaction vessel.

12. A method as claimed in claim 10 when appendant to claim 7 or claim 8 in which the polypeptide is recovered and purified from culture medium token from the cell-free region of the reaction vessel.

13. A method as claimed in any of claims 10 to 12 in which the DNA coding for the polypeptide is under the control of an inducible promoter, the promoter being induced by a component of the serum-free medium.

14. A method as claimed in any of claims 10 to 13 in which the mammalian cells are a continuous cell line.

**15.** A method as claimed in claim 14 in which the continuous cell line is Chinese hamster ovary cell line.

**16.** A method as claimed in any of claims 10 to 15 in which the polypeptide is human growth hormones.

**17.** A method as claimed in any one of claims 1 to 16 in which the serum-free medium is also protein-free.

**Patentansprüche**

**1.** Verfahren zum Kultivieren von Säugerzellen, die ein Anhaften erfordern, umfassend die folgenden Schritte:

1. Kultivieren der Säugerzellen in einem Serum-enthaltenden Medium in einem Reaktionsgefäß, das mit einem Trägermedium ausgerüstet ist, an das die Zellen anhaften werden, bis die Zellen daran anhaften und bis zur gewünschten Zell-Konzentration gezüchtet worden sind;
2. Dekantieren des Serum-enthaltenden Mediums;
3. Ersetzen des dekantierten Mediums mit einem Serum-freien Medium, das in Lösung ein nicht-toxisches Polymer enthält, das die Film-Entwässerung um die Zellen und das Träger-Medium herum reduziert; und
4. direktes Belüften der Kultur.

**2.** Verfahren nach Anspruch 1, bei dem das in dem Serum-freien Medium vorhandene nicht-toxische Polymer aus der Gruppe bestehend aus Polyethylenglykol und Polyvinylpyrrolidon ausgewählt ist.

**3.** Verfahren nach Anspruch 1, bei dem das in dem Serum-freien Medium vorhandene nicht-toxische Polymer ein Polymer ist, das eine oder mehrere Alkylenoxide enthält.

**4.** Verfahren nach Anspruch 3, bei dem das Alkylenoxid 2 oder 3 Kohlenstoffatome enthält.

**5.** Verfahren nach Anspruch 4, bei dem das Polymer ein Copolymer aus Propylenoxid und Ethylenoxid ist.

**6.** Verfahren nach Anspruch 3, bei dem das Polymer der Alkylenoxide im Medium in einer Konzentration von 0,1 bis 1,0 Gew.-% vorhanden ist.

**7.** Verfahren nach irgendeinem der vorhergehenden Ansprüche, bei dem das Reaktionsgefäß in einen Kultur-Bereich und einen Zell-freien Bereich aufgeteilt wird, wobei der Kultur-Bereich von dem Zell-freien Bereich mit Hilfe eines Filters abgetrennt wird, durch welches Kulturmedium aber keine Zellen durchgeleitet werden kann.

**8.** Verfahren nach Anspruch 7, bei dem die Belüftung der Kultur durch Hineinperlen eines Gases in den Zell-freien Bereich des Reaktionsgefäßes durchgeführt wird.

**9.** Verfahren nach irgendeinem der vorhergehenden Ansprüche, bei dem die Belüftung mit Hilfe eines im Reaktionsgefäß vorhandenen Airlifts durchgeführt wird.

**10.** Verfahren nach irgendeinem der vorhergehenden Ansprüche, bei dem die Säugerzelle gentechnisch verändert wurde, um eine für ein Polypeptid kodierende DNA zu enthalten, damit das Wachstum der Zellen zu einer Produktion des Polypeptids führt.

**11.** Verfahren nach Anspruch 10, bei dem das Polypeptid aus dem kontinuierlich aus dem Reaktionsgefäß entfernten Kulturmedium gewonnen wird und Serum-freies Medium kontinuierlich dem Reaktionsgefäß zugegeben wird.

**12.** Verfahren nach Anspruch 10, bei dem, wenn von Anspruch 7 oder Anspruch 8 abhängig, das Polypeptid aus dem Kulturmedium gewonnen und gereinigt wird, das aus dem Zell-freien Bereich des Reaktionsgefäßes entnommen wird.

**13.** Verfahren nach irgendeinem der Ansprüche 10 bis 12, bei dem die für das Polypeptid kodierende DNA unter Kontrolle eines induzierbaren Promotors ist, wobei der Promotor durch eine Komponente des Serum-freien Mediums induziert wird.

EP 0 318 487 B1

**14.** Verfahren nach irgendeinem der Ansprüche 10 bis 13, bei dem die Säugerzellen eine kontinuierliche Zellinie sind.

**15.** Verfahren nach Anspruch 14, bei dem die kontinuierliche Zellinie eine Chinesische Hamster-Ovarzellinie ist.

**16.** Verfahren nach irgendeinem der Ansprüche 10 bis 15, bei dem das Polypeptid ein humanes Wachstumshormon ist.

**17.** Verfahren nach irgendeinem der Ansprüche 1 bis 16, bei dem das Serum-freie Medium ferner Protein-frei ist.

**Revendications**

**1.** Méthode de culture de cellules de mammifère qui nécessite une fixation, comprenant les étapes suivantes :
- 1. Mise en culture des cellules de mammifère dans un milieu contenant du sérum dans un récipient réactionnel pourvu d'un milieu de support auquel les cellules s'attacheront jusqu'à ce que les cellules se soient attachées et se soient développer jusqu'à la concentration souhaité de cellules ;
- 2. Décantation du milieu contenant du sérum ;
- 3. Remplacement du milieu décanté par un milieu sans sérum qui contient en solution un polymère non toxique qui réduit le drainage du film autour des cellules et du milieu de support ; et
- 4. Mise de la culture à une aération directe.

**2.** Méthode selon la revendication 1, où le polymère non toxique présent dans le milieu sans sérum est choisi dans le groupe comprenant du polyéthylène glycol et de la polyvinyl pyrrolidone.

**3.** Méthode selon la revendication 1, où le polymère non toxique présent dans le milieu sans sérum est un polymère qui contient un ou plusieurs oxydes d'alkylène.

**4.** Méthode selon la revendication 3, où l'oxyde d'alkylène contient 2 ou 3 atomes de carbone.

**5.** Méthode selon la revendicaiton 4, où le polymère est un copolymère d'oxyde de propylène et d'oxyde d'éthylène.

**6.** Méthode selon la revendicaiton 3, où le polymère d'oxydes d'alkylène est présent dans le milieu à une concentration de 0,1 à 1,0% en poids.

**7.** Méthode selon toute revendication précédente, où le récipient réactionnel est divisé en une région de culture et une région sans cellule , la région de culture étant séparée de la région sans cellule au moyen d'un filtre par où le milieu de culture peut passer mais non pas les cellules.

**8.** Méthode selon la revendication 7, où l'aération de la culture est entreprise en faisant barboter un gaz dans la région sans cellule du récipient réactionnel.

**9.** Méthode selon toute revendication précédente, où l'aération est effectuée au moyen d'un décollage au gaz présent dans le récipient réactionnel.

**10.** Méthode selon toute revendication précédente, où la cellule de mammifère est génétiquement produite pour contenir un ADN codant un polypeptide de manière que la croissance des cellules donne la production du polypeptide.

**11.** Méthode selon la revendication 10, où le polypeptide est récupéré du milieu de culture continuellement enlevé du récipient réactionnel et du milieu frais sans sérum est continuellement ajouté au récipient réactionnel.

9

**12.** Méthode selon la revendication 10, dépendante de la revendication 7 ou de la revendication 8, où le polypeptide est récupéré et purifié du milieu de culture pris de la région sans cellule du récipient réactionnel.

**13.** Méthode selon l'une des revendications 10 à 12, où l'ADN codant pour le polypeptide est sous le contrôle d'un promoteur inductible, le promoteur étant induit par un composant du milieu sans sérum.

**14.** Méthode selon toute revendication 10 à 13, où les cellules de mammifère sont une lignée continue de cellules.

**15.** Méthode selon la revendication 14, où la lignée continue de cellules est une lignée de cellules d'ovaire de hamster chinois.

**16.** Méthode selon toute revendication 10 à 15, où le polypeptide est l'hormone de la croissance humaine.

**17.** Méthode selon toute revendication 1 à 16, où le milieu sans sérum est également sans protéine.

Figure 1